# EUROPEAN PATENT SPECIFICATION

(11) **EP 3 235 484 B1**
(45) Date of publication and mention of the grant of the patent: **13.10.2021**
(21) Application number: 16197637.8
(22) Date of filing: 08.11.2016
(51) Int. Cl.: A61H 7/00, G16H 20/30

(54) **METHOD AND DEVICE FOR CONTROLLING TARGET MASSAGE EQUIPMENT**
VERFAHREN UND VORRICHTUNG ZUR STEUERUNG VON ZIELMASSAGEAUSRÜSTUNG
PROCÉDÉ ET DISPOSITIF DE COMMANDE D'ÉQUIPEMENT DE MASSAGE CIBLÉ

(30) Priority: 21.04.2016 CN 201610252504
(43) Date of publication of application: 25.10.2017
(73) Proprietor: Beijing Xiaomi Mobile Software Co., Ltd., Beijing 100085 (CN)
(72) Inventor: LI, Junjie, Beijing, 100085 (CN); WANG, Gang, Beijing, 100085 (CN); ZHANG, Xiangyang, Beijing, 100085 (CN)
(74) Representative: Cabinet Beau de Loménie

(56) References cited:
- KR-A- 20030 035 048
- US-A1- 2005 113 723
- US-A1- 2008 045 384

## Description

### FIELD

The present invention relates to a field of massage equipment technology, and more particularly relates to a method and a device for controlling a target massage equipment.

### BACKGROUND

At present, with the development of society, an increasing number of users have better fitness consciousness, and an effective recovery after exercises often brings a better effect. Thus, massage equipment is required to provide massage for assisting the recovery. However, the existing massage equipment is not intelligent enough, and the massage mode is simplex, thereby causing great inconvenience for the user.

US2005/0113723A1 discloses a calorie meter which is attachable to a human body measures the acceleration of its attached part, and a CPU estimates activity information of the human body based on the measured acceleration.

### SUMMARY

Embodiments of the present invention provide a method and a device for controlling a target massage equipment in accordance with the claims.

It should be understood that the above general descriptions and the following detail descriptions are explanatory and illustrative, and these descriptions shall not be construed to limit the present invention.

### BRIEF DESCRIPTION OF THE DRAWINGS

The accompanying drawings, which are incorporated in and constitute a part of this specification, illustrate embodiments consistent with the present invention and, together with the description, serve to explain the principles of the present invention.
Fig. 1 is a flow chart of a method for controlling a target massage equipment according to an example embodiment of the present invention.
Fig. 2 is a flow chart of obtaining exercise information according to an example embodiment of the present invention.
Fig. 3 is another flow chart of obtaining exercise information according to an example embodiment of the present invention.
Fig. 4 is yet another flow chart of obtaining exercise information according to an example embodiment of the present invention.
Fig. 5 is a flow chart of controlling a target massage equipment to operate according to exercise information, according to an example embodiment of the present invention.
Fig. 6 is another flow chart of controlling a target massage equipment to operate according to exercise information, according to an example embodiment of the present invention.
Fig. 7 is a block diagram of a device for controlling a target massage equipment according to an example embodiment of the present invention.
Fig. 8 is a block diagram of an obtaining module according to an example embodiment of the present invention.
Fig. 9 is a block diagram of another obtaining module according to an example embodiment of the present invention.
Fig. 10 is a block diagram of yet another obtaining module according to an example embodiment of the present invention.
Fig. 11 is a block diagram of a control module according to an example embodiment of the present invention.
Fig. 12 is a block diagram of another control module according to an example embodiment of the present invention.
Fig. 13 is a block diagram of a device for controlling a target massage equipment according to an example embodiment of the present invention.

### DETAILED DESCRIPTION

Reference will now be made in detail to exemplary embodiments, examples of which are illustrated in the accompanying drawings. The following description refers to the accompanying drawings in which the same numbers in different drawings represent the same or similar elements unless otherwise represented. The implementations set forth in the following description of exemplary embodiments do not represent all implementations consistent with the invention. Instead, they are merely examples of devices and methods consistent with aspects related to the invention as recited in the appended claims.

At present, with the development of society, an increasing number of users have better fitness consciousness, and an effective recovery after exercises often brings a better effect. Thus, massage equipment is required to provide massage to assist the recovery. However, the existing massage equipment is not intelligent enough, and the massage mode is simplex, thereby causing great inconvenience for users.

In order to solve the above problems, embodiments of the present invention provide a method for controlling a target massage equipment. The method may be applied in a control program, a system or a device of the target massage equipment, and the executive subject corresponding the method may be a terminal or a target massage equipment.

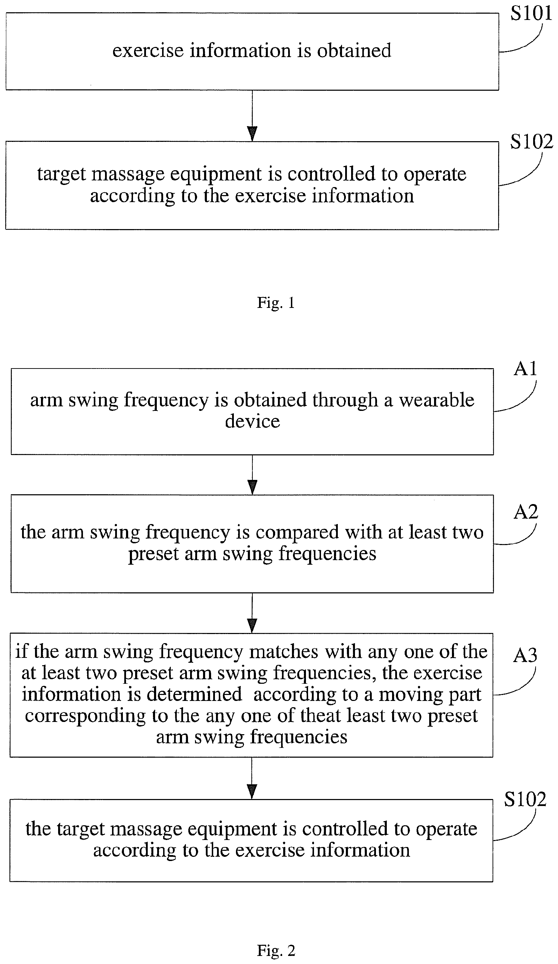

Fig. 1 is a flow chart of a method for controlling a target massage equipment according to an example embodiment of the present invention.

As shown in Fig. 1, the method may include: act S101 to act S102.

In act S101, exercise information is obtained.

The exercise information refers to information obtained when a user is doing physical exercise (or keeping fit) within a preset time period (such as a latest week or latest N days). The exercise information includes but is not limited to at least one of the followings: body moving part information of the user, an exercise intensity ratio between each two moving parts, exercise intensity information of each moving part and information of a used exercise equipment.

Additionally, the executive subject for obtaining the exercise information may be a terminal or a target massage equipment.

In act S102, the target massage equipment is controlled to operate according to the exercise information. When the target massage equipment is controlled to operate according to the exercise information, the target massage equipment may provide personalized massage service for the user automatically according to the exercise information, or the target massage equipment may be controlled to move through the terminal so as to provide personalized massage service for the user.

The user's exercise status may be precisely determined according to the obtained exercise information, such that the target massage equipment may be controlled to conduct a targeted movement based on the exercise information when the user is doing exercise, and to automatically provide the most comfortable, the most suitable and the most effective massage for the user with respect to the user's exercise information, such that the user's body may be recovered better and faster through the optimum massage after exercises, thereby prevent the user from selecting the massage mode manually and reducing the user's operations.

Fig. 2 is a flow chart of obtaining exercise information according to an example embodiment of the present invention.

As shown in Fig. 2, in an embodiment, the act S101 in Fig. 1 may be executed as follows.

In act A1, an arm swing frequency is obtained through a wearable device, in which the wearable device includes at least one of a smart band and a smart watch.

To obtain the exercise information by either a terminal or the target massage equipment, the arm swing frequency may be obtained firstly through a wearable device, in which the wearable device may be a smart band and a smart watch wore on the user's wrist. The wearable device, of course, may also be a smart band wore on the user's finger. These wearable devices may be connected to the terminal or the target massage equipment through a communication mode such as blue-tooth, or even infrared technology, WiFi (Wireless Fidelity, wireless local area network based on IEEE 802.11b standard), and then the swing frequency is transmitted to the terminal or the target massage equipment, such that the terminal or the target massage equipment may perform operations shown in the following act A2 and act A3.

In act A2, the arm swing frequency is compared with at least two preset arm swing frequencies.

In act A3, if the arm swing frequency matches with any one of the at least two preset arm swing frequencies, the exercise information is determined according to a moving part of a user's body corresponding to the any one of the at least two preset arm swing frequencies.

Since each pre-stored preset arm swing frequency may be corresponding to a distinct moving part (for example, when a user is lifting dumbbells, the arm swing frequency and the moving part are different from those when the user is jogging), each preset arm swing frequency may correspond to distinct exercise information. Thus, it may be judged whether the arm swing frequency obtained in real-time matches with one of the at least two preset arm swing frequencies by comparing the obtained arm swing frequency with the at least two preset arm swing frequencies. If the arm swing frequency obtained in real-time matches with any one of the at least two preset arm swing frequencies (for example, the arm swing frequency obtained in real-time is equal to any one of the at least two preset arm swing frequencies completely, or the difference between the two swing frequencies is less than or equal to a preset frequency threshold), it indicates that the exercise information corresponding to the arm swing frequency obtained in real-time is the same as the exercise information corresponding to the preset arm swing frequency matched with the arm swing frequency obtained in real-time. Therefore, the current exercise information of the user may be determined according to the moving part corresponding to the preset arm swing frequency matched with the arm swing frequency obtained in real-time. For example, the user's body moving part information or the like may be determined according to the moving part corresponding to the preset arm swing frequency matched with the arm swing frequency obtained in real-time.

Besides, since the arm swing frequency is not often at a constant speed no matter what kind of equipment is used or what kind of exercise intensity is applied during the user's exercise or fitness process, each of the at least two preset arm swing frequencies may have frequency fluctuation or even may be an arm swing frequency spectrogram, also the arm swing frequency obtained in real-time may have frequency fluctuation or even may be an arm swing frequency spectrogram. Thus, when comparing, the similarity of two arm swing frequency spectrograms may be compared. If the similarity is higher than a preset similarity (e.g. 90%), the exercise information may be determined according to a preset swing frequency spectrogram with which the arm swing frequency obtained in real-time has a similarity higher than the preset similarity.

Additionally, each of the at least two preset arm swing frequencies may include an arm swing frequency in the vertical direction and an arm swing frequency in the horizontal direction. When comparing, the arm swing frequency in each direction may be compared respectively. If the arm swing frequency in each direction is matched respectively, the exercise information may be determined according to the moving part corresponding to the preset arm swing frequency matched, thereby increasing accuracy of the determination result.

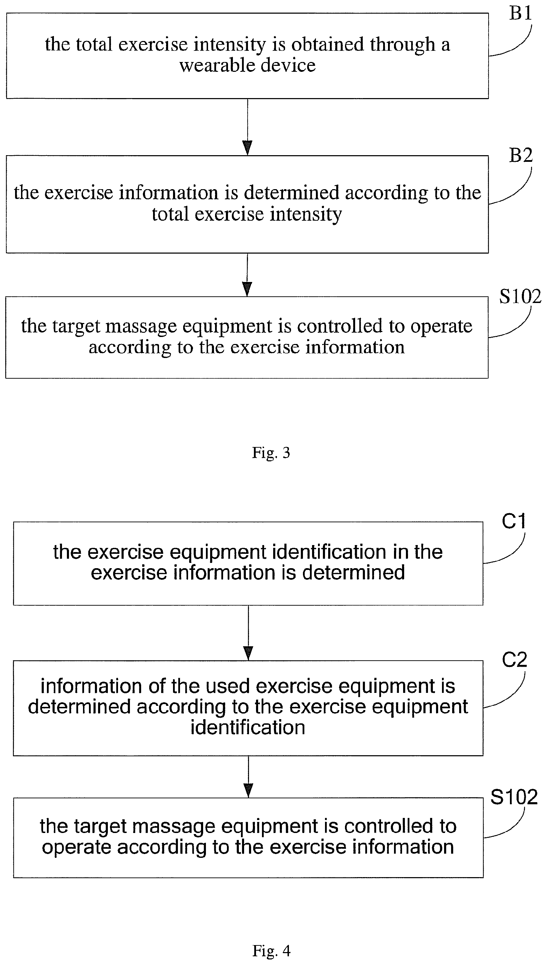

Fig. 3 is another flow chart of obtaining exercise information according to an example embodiment of the present invention.

As shown in Fig. 3, in an embodiment, the act S101 in Fig. 1 may be executed as follows.

In act B1, a total exercise intensity is obtained through a wearable device, in which the total exercise intensity includes one or more of a pulse rate, a pulse intensity, a heartbeat frequency and a heartbeat intensity when the user is doing exercise.

The wearable device such as a smart band and a smart watch may directly detect the pulse rate and the pulse intensity when the user is doing exercise, and then the heartbeat frequency and the heartbeat intensity of the user may be obtained indirectly according to the pulse rate and the pulse intensity, such that the total exercise intensity when the user is doing exercise may be obtained through the wearable device. Further, these wearable devices may be connected to the terminal or the target massage equipment through a communication mode such as blue-tooth, or even infrared technology, WiFi (Wireless Fidelity, wireless local area network based on IEEE 802.11b standard), and then the total exercise intensity is transmitted to the terminal or the target massage equipment, in order to enable the terminal or the target massage equipment to perform operations shown in the following act B2 and act B3.

In act B2, the exercise information is determined according to the total exercise intensity.

After the total exercise intensity is obtained, the terminal or the target massage equipment may determine the current exercise information of the user automatically according to the total exercise intensity. For example, the exercise intensity ratio between various moving parts and the exercise intensity information of each moving part may be determined accurately by combining with the body moving part information obtained based on the arm swing frequency (for example, after the user's body moving part is determined as the leg according to the arm swing frequency and the user is perhaps using a running machine for jogging, the exercise intensities of upper limbs and lower limbs may be determined according to the total exercise intensity, and the exercise intensity ratio of upper limbs to lower limbs may be further determined, etc.). Thus, the target massage equipment may conduct a targeted movement based on the exercise information when the user is doing exercise, so as to automatically provide the most comfortable, the most suitable and the most effective massage for the user with respect to the user's exercise information. Therefore, after exercises, the user's body may be recovered better through the optimum massage, thereby prevent the user from selecting the massage mode manually and reducing the user's operations.

Additionally, this embodiment may be combined with the above embodiment in which the exercise information is determined according to the arm swing frequency obtained by the wearable device or may also be combined with the following embodiment in which information of an exercise equipment used when the user is doing exercise is determined, so as to obtain more comprehensive and accurate exercise information.

Fig. 4 is another flow chart of obtaining exercise information according to an example embodiment of the present invention.

As shown in Fig. 4, in an embodiment, the act S101 in Fig. 1 may be executed as follows.

In act C1, an exercise equipment identification in the exercise information is determined.

The terminal or the target massage equipment may identify the exercise information obtained by the wearable device, and then determine the exercise equipment identification included in the exercise information. The specific method for obtaining the exercise equipment identification by the wearable device includes but is not limited to the followings.

The user manually inputs the exercise equipment identification of the exercise equipment used when the user is doing exercise into the wearable device such as the smart band, the smart watch etc. worn by himself/herself.

Alternatively, the user's current movement direction may be determined automatically by an acceleration sensor or a direction sensor embedded in the wearable device such as the smart band, the smart watch etc. worn by the user, and then the exercise equipment identification may be determined indirectly. For example, if the acceleration sensor or the direction sensor detects that the user's arm moves upwardly all the time, the exercise equipment identification may be determined as an identification of a dumbbell or a horizontal bar.

Alternatively, if the exercise equipment used by the user is relatively intelligent (such as configured with blue-tooth functionality), the exercise equipment identification may be sent to the wearable device the user wears such as the smart band, the smart watch etc. via the blue tooth.

In act C2, information of the used exercise equipment may be determined according to the exercise equipment identification, in which the information of the used exercise equipment includes: at least one of a name of the exercise equipment and a working parameter of the exercise equipment.

After the exercise equipment identification is determined by the terminal or the target massage equipment, the information of the exercise equipment used when the user is doing exercise, such as the name of the exercise equipment and the working parameter of the exercise equipment (such as movement rate of the running machine if the used exercise equipment is a running machine, or weight of the dumbbell if the used exercise equipment is a dumbbell) etc., may be determined according to the exercise equipment identification. Then, the target massage equipment may be controlled to provide a targeted massage for the user according to the information of the exercise equipment used when the user is doing exercise, such that the user's body may be recovered from fatigue as soon as possible, and the user's comfortable experience may be improved.

Additionally, this embodiment may be combined with the above embodiment in which the exercise information is determined according to the arm swing frequency obtained by the wearable device, or may also be combined with the above embodiment in which the exercise information is determined according to the obtained total exercise intensity, so as to obtain more comprehensive and accurate exercise information.

In an embodiment, the exercise information includes at least one of the followings: body moving part information, an exercise intensity ratio between each two moving parts, exercise intensity information of each moving part and information of the used exercise equipment.

The exercise information includes but is not limited to at least one of the followings: the user's body moving part information, an exercise intensity ratio between various moving parts, exercise intensity information of each moving part and the information of the used exercise equipment. The exercise information, for example, may also include total exercise time of the user.

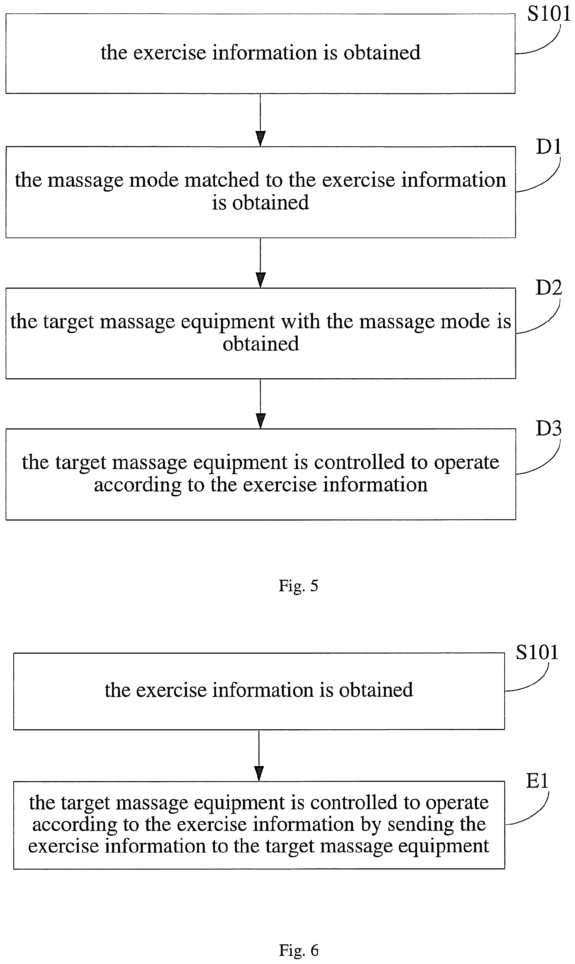

Fig. 5 is a flow chart of controlling a target massage equipment to operate according to exercise information, according to an example embodiment of the present invention.

As shown in Fig. 5, in an embodiment, the act S102 in Fig. 1 may be executed as follows.

In act D1, a massage mode matched with the exercise information is determined.

When the target massage equipment is controlled to operate according to the exercise information for providing a targeted massage for a user, a massage mode matched with the exercise information may be obtained firstly, i.e., the massage mode which matches with the exercise information and make the user's body be recovered from the fatigue due to exercise as soon as possible is obtained. For example, if the exercise equipment used by the user is a running machine, since the main body moving part is the leg and the leg conducts a large amount of activity, the massage mode may be a foot massage mode, a leg massage mode etc.; if the exercise equipment used by the user is a dumbbell or a horizontal bar, since the main body moving part is the arm and the arm conducts a large amount of activity, the massage mode may be an arm massage mode, a hand massage mode etc..

In act D2, the target massage equipment with the massage mode is obtained.

For example, when the massage mode is foot massage mode, the target massage equipment may be a massage chair or a foot massager, a foot massage basin etc..

In act D3, the target massage equipment is controlled to operate based on the massage mode.

Further, the target massage equipment with the massage mode is obtained, and then the target massage equipment is controlled to operate based on the massage mode so as to automatically provide the most comfortable, the most suitable and the most effective massage for the user with respect to the user's exercise information, such that the user's body may be recovered better and faster through the optimum massage after exercises, thereby prevent the user from selecting the massage mode manually and reducing the user's operations.

Since each massage mode has its corresponding preset massage parameter but each user's specific exercise information is distinct (for example, when the same exercise equipment is used to do exercise, the total exercise intensity, the exercise intensity of each body part, exercise ratio and length of exercise time may be changed), after the rough matched massage mode is determined, the target massage equipment may be controlled to operate followings.

The exercise information is compared with the preset exercise information corresponding to the massage mode.

The preset massage parameter corresponding to the massage mode is adjusted according to the comparison result, in which the preset massage parameter includes: one or more of a preset length of massage time and a preset massage intensity. For example, when the massage mode is upper limb massage, and the exercise intensity ratio of the upper limb to the lower limb in this exercise is higher than the preset exercise intensity ratio of the upper limb to the lower limb corresponding to the massage mode, the preset massage parameter corresponding to the massage mode may be increased for increasing the massage time and intensity for the upper limb; when the exercise intensity ratio of the upper limb to the lower limb in this exercise is lower than the preset exercise intensity ratio of the upper limb to the lower limb corresponding to the massage mode, the preset massage parameter corresponding to the massage mode may be decreased for decreasing the massage time and intensity for the upper limb. In this way, the inherent preset massage parameter of the massage mode may be adjusted according to the user's specific exercise information, so as to provide a more intelligent targeted massage service for the user automatically. Thus, the user's body may be recovered from exercise better and faster, and the user's comfortable experience may be improved further.

In an embodiment, the massage mode includes: a historical massage mode matched with the exercise information.

When determining the massage mode, it is unnecessary to re-determine the mode every time. The exercise information of the user in each exercise and the determined matched massage mode may be stored in relation to each other. Thus, when the user does exercise next time, if the determined exercise information is exactly the same as that in one previous exercise, or the difference is minor, it is not required to re-determine the massage mode suitable for the user. Instead, the historical massage mode matched with the exercise information in this exercise may be directly determined as the massage mode for this exercise, so as to avoid re-determination and reduce operations and burden of the terminal and the target massage equipment.

Fig. 6 is another flow chart of controlling a target massage equipment to operate according to exercise information, according to an example embodiment of the present invention.

As shown in Fig. 6, in an embodiment, the act S102 in Fig. 1 may be executed as follows.

In act E1, the target massage equipment is controlled to operate according to the exercise information by sending the exercise information to the target massage equipment.

When the subject of obtaining the exercise information is a terminal and the target massage equipment is controlled to operate according to the exercise information, the terminal may send the obtained exercise information to the target massage equipment, and then control the target massage equipment to operate according to the exercise information, such that the target massage equipment is controlled automatically through the terminal to provide the most comfortable, the most suitable and the most effective massage for the user with respect to the user's exercise information. Thus, after exercises, the user's body may be recovered better through the optimum massage, thereby prevent the user from selecting the massage mode manually and reducing the user's operations.

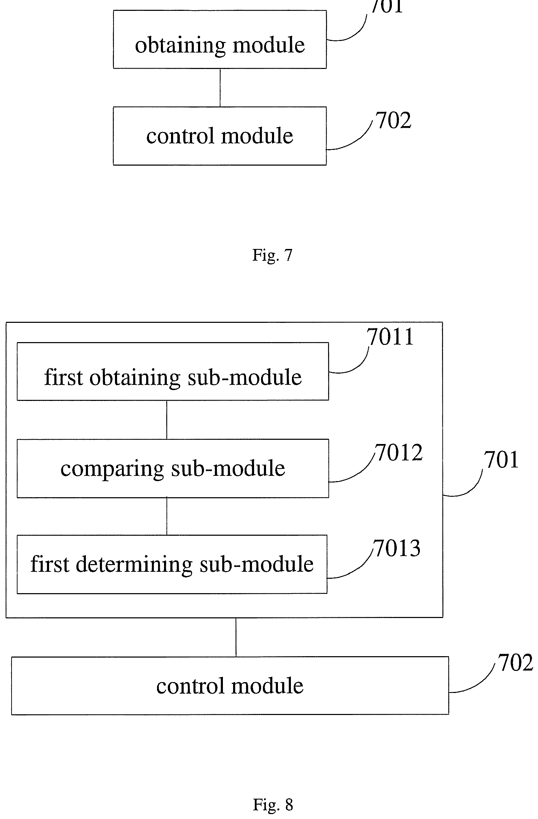

Corresponding to the above method for controlling a target massage equipment provided by embodiments of the present invention, embodiments of the present invention also provide a device for controlling a target massage equipment. Fig. 7 is a block diagram of a device for controlling a target massage equipment according to an example embodiment of the present invention.

As shown in Fig. 7, the device includes: an obtaining module 701 and a control module 702. The obtaining module 701 is configured to obtain exercise information.

The exercise information obtained by the obtaining module 701 is information obtained when the user is doing exercise this time. The exercise information includes but is not limited to at least one of the followings: the user's body moving part information, an exercise intensity ratio between each two moving parts, exercise intensity information of each moving part and information of a used exercise equipment.

Additionally, the subject of obtaining the exercise information may be a terminal or a target massage equipment.

The control module 702 is configured to control the target massage equipment to operate according to the exercise information obtained by the obtaining module 701. When the target massage equipment is controlled to operate according to the exercise information, the target massage equipment may provide personalized massage service for the user automatically, or the target massage equipment may be controlled to operate through the terminal so as to provide personalized massage service for the user.

The user's exercise status may be precisely determined according to the obtained exercise information. Thus, the control module 702 may control the target massage equipment to conduct a targeted movement based on the exercise information when the user is doing exercise, so as to automatically provide the most comfortable, the most suitable and the most effective personalized massage for the user with respect to the user's exercise information. Therefore, after exercises, the user's body may be recovered better through the optimum massage, thereby prevent the user from selecting the massage mode manually and reducing the user's operations.

Fig. 8 is a block diagram of an obtaining module according to an example embodiment of the present invention.

As shown in Fig. 8, in an embodiment, the obtaining module 701 in Fig. 7 may include: a first obtaining sub-module 7011, a comparing sub-module 7012, and a first determining sub-module 7013.

The first obtaining sub-module 7011 is configured to obtain an arm swing frequency through a wearable device, in which the wearable device includes at least one of a smart band and a smart watch.

To obtain the exercise information by either a terminal or the target massage equipment, the arm swing frequency may be obtained firstly by the first obtaining sub-module 7011 through a wearable device, in which the wearable device may be a smart band and a smart watch worn on the user's wrist. The wearable device, of course, may also be a smart band wore on the user's finger. These wearable devices may be connected to the terminal or the target massage equipment through a communication mode such as blue-tooth, or even infrared technology, WiFi (Wireless Fidelity, wireless local area network based on IEEE 802.11b standard), and then the swing frequency is transmitted to the terminal or the target massage equipment, in order to enable the terminal or the target massage equipment to perform operations.

The comparing sub-module 7012 is configured to compare the arm swing frequency obtained by the first obtaining sub-module 7011 with at least two preset arm swing frequencies.

The first determining sub-module 7013 is configured to determine the exercise information according to a moving part of a user's body corresponding to any one of the at least two preset arm swing frequencies when the comparison result obtained by the comparing sub-module 7012 indicates that the arm swing frequency matches with the any one of the at least two preset arm swing frequencies.

Since each pre-stored preset arm swing frequency may correspond to a distinct moving part (for example, when a user is lifting dumbbells, the arm swing frequency and the moving part are different from those when the user is jogging), each preset arm swing frequency may correspond to distinct exercise information. Thus, it may be judged whether the arm swing frequency may obtained in real-time matches with one of the at least two preset arm swing frequencies by comparing the obtained arm swing frequency with the at least two preset arm swing frequencies by the comparing sub-module 7012. If the determination result of the first determining sub-module 7013 is that the arm swing frequency obtained in real-time matches with any one of the at least two preset arm swing frequencies (for example, the arm swing frequency obtained in real-time is equal to any one of the at least two preset arm swing frequencies completely, or the difference between the two swing frequencies is less than or equal to a preset frequency threshold), it indicates that the exercise information corresponding to the arm swing frequency obtained in real-time is the same as the exercise information based on the preset arm swing frequency matched with the arm swing frequency obtained in real-time. Therefore, the current exercise information of the user may be determined according to the moving part corresponding to the preset arm swing frequency matched with the arm swing frequency obtained in real-time. For example, the user's body moving part information or the like may be determined according to the moving part corresponding to the preset arm swing frequency matched with the arm swing frequency obtained in real-time.

Besides, since the arm swing frequency is not often at a constant speed no matter what kind of equipment is used or what kind of exercise intensity is applied during the user's exercise or fitness process, each of the at least two preset arm swing frequencies may have frequency fluctuation or even may be an arm swing frequency spectrogram, also the arm swing frequency obtained in real-time may have frequency fluctuation or even may be an arm swing frequency spectrogram. Thus, when comparing, the similarity of two arm swing frequency spectrograms may be compared. If the similarity is higher than a preset similarity (e.g. 90%), the exercise information may be determined according to a preset swing frequency spectrogram with which the arm swing frequency obtained in real-time has a similarity higher than the preset similarity.

Additionally, each of the at least two preset arm swing frequencies may include an arm swing frequency in the vertical direction and an arm swing frequency in the horizontal direction. When comparing, the arm swing frequency in each direction may be compared respectively. If the arm swing frequency in each direction is matched respectively, the exercise information may be determined according to the moving part corresponding to the preset arm swing frequency matched, thereby increasing accuracy of the determination result.

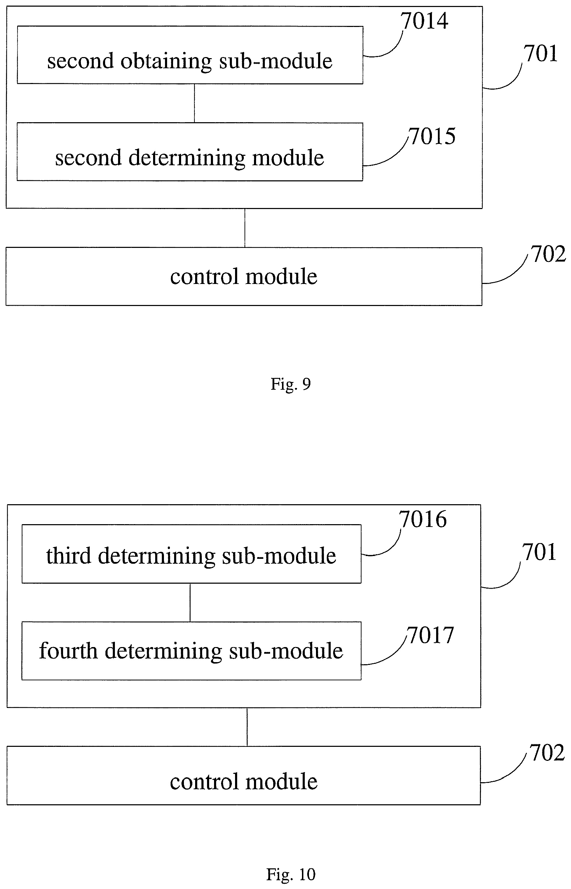

Fig. 9 is a block diagram of another obtaining module according to an example embodiment of the present invention.

As shown in Fig. 9, in an embodiment, the obtaining module 701 in Fig. 7 may include: a second obtaining sub-module 7014, and a second determining sub-module 7015.

The second obtaining sub-module 7014 is configured to obtain a total exercise intensity through a wearable device, in which the total exercise intensity includes one or more of a pulse rate, a pulse intensity, a heartbeat frequency and a heartbeat intensity when the user is doing exercise.

The wearable device such as a smart band and a smart watch may directly detect the pulse rate and the pulse intensity when the user is doing exercise, and then the heartbeat frequency and the heartbeat intensity of the user may be obtained indirectly according to the pulse rate and the pulse intensity, such that the second obtaining sub-module 7014 may obtain the total exercise intensity when the user is doing exercise through the wearable device. Further, these wearable devices may be connected to the terminal or the target massage equipment through a communication mode such as blue-tooth, or even infrared technology, WiFi (Wireless Fidelity, wireless local area network based on IEEE 802.11b standard), and then the total exercise intensity is transmitted to the terminal or the target massage equipment, in order to enable the terminal or the target massage equipment to perform operations.

The second determining sub-module 7015 is configured to determine the exercise information according to the total exercise intensity obtained by the second obtaining sub-module 7014.

After the total exercise intensity is obtained by the second obtaining sub-module 7014, the second determining sub-module 7015 in the terminal or the target massage equipment may determine the current exercise information of the user automatically according to the total exercise intensity. For example, the exercise intensity ratio between various moving parts and the exercise intensity information of each moving part may be determined accurately by combining with the body moving part information obtained based on the arm swing frequency (for example, after the user's body moving part is determined as the leg according to the arm swing frequency and the user is perhaps using a running machine for jogging, the exercise intensities of upper limbs and lower limbs may be determined according to the total exercise intensity, and the exercise intensity ratio of upper limbs to lower limbs may be further determined, etc.). Thus, the target massage equipment may conduct a targeted movement based on the exercise information when the user is doing exercise, so as to automatically provide the most comfortable, the most suitable and the most effective massage for the user with respect to the user's exercise information. Therefore, after exercise, the user's body may be recovered better through the optimum massage, thereby prevent the user from selecting the massage mode manually and reducing the user's operations.

Additionally, this embodiment may be combined with the above embodiment in which the exercise information is determined according to the arm swing frequency obtained by the wearable device, or may also be combined with the following embodiment in which information of an exercise equipment used when the user is doing exercise is determined, so as to obtain more comprehensive and accurate exercise information.

Fig. 10 is a block diagram of another obtaining module according to an example embodiment of the present invention.

As shown in Fig. 10, in an embodiment, the obtaining module 701 in Fig. 7 may include: a third determining sub-module 7016, and a fourth determining sub-module 7017.

The third determining sub-module 7016 is configured to determine an exercise equipment identification in the exercise information.

The third determining sub-module 7016 in the terminal or the target massage equipment may identify the exercise information obtained by the wearable device, and then determine the exercise equipment identification included in the exercise information. The specific method for obtaining the exercise equipment identification by the wearable device includes but is not limited to the followings.

The user manually inputs the exercise equipment identification of the exercise equipment used when the user is doing exercise into the wearable device such as the smart band, the smart watch etc. worn by himself/herself.

Alternatively, the user's current movement direction may be determined automatically by an acceleration sensor or a direction sensor embedded in the wearable device such as the smart band, the smart watch etc. worn by the user, and then the exercise equipment identification may be determined indirectly. For example, if the acceleration sensor or the direction sensor detects that the user's arm moves upwardly all the time, the exercise equipment identification may be determined as an identification of a dumbbell or a horizontal bar.

Alternatively, if the exercise equipment used by the user used is relatively intelligent (such as configured with blue-tooth functionality), the exercise equipment identification may be sent to the wearable device the user wears such as the smart band, the smart watch etc. via the blue-tooth.

The fourth determining sub-module 7017 is configured to determine information of the used exercise equipment according to the exercise equipment identification determined by the third determining sub-module 7016, in which the information of the used exercise equipment includes: at least one of a name of the exercise equipment and a working parameter of the exercise equipment.

After the exercise equipment identification is determined by the third determining sub-module 7016 in the terminal or the target massage equipment, the fourth determining sub-module 7017 may determine the information of the exercise equipment used when the user is doing exercise, such as the name of the exercise equipment and the working parameter of the exercise equipment (such as movement rate of the running machine if the used exercise equipment is a running machine, or weight of the dumbbell if the used exercise equipment is a dumbbell) etc.. Then, the target massage equipment may be controlled to provide a targeted massage for the user according to the information of the exercise equipment used when the user is doing exercise, such that the user's body may be recovered from fatigue as soon as possible, and the user's comfortable experience may be improved.

Additionally, this embodiment may be combined with the above embodiment in which the exercise information is determined according to the arm swing frequency obtained by the wearable device, or may be combined with the above embodiment in which the exercise information is determined according to the obtained total exercise intensity, so as to obtain more comprehensive and accurate exercise information.

In an embodiment, the exercise information includes at least one of the followings: body moving part information, an exercise intensity ratio between each two moving parts, exercise intensity information of each moving part and information of a used exercise equipment.

The exercise information includes but is not limited to at least one of the followings: body moving part information of the user, an exercise intensity ratio between various moving parts, exercise intensity information of each moving part and the information of the used exercise equipment. The exercise information, for example, may also include total exercise time of the user.

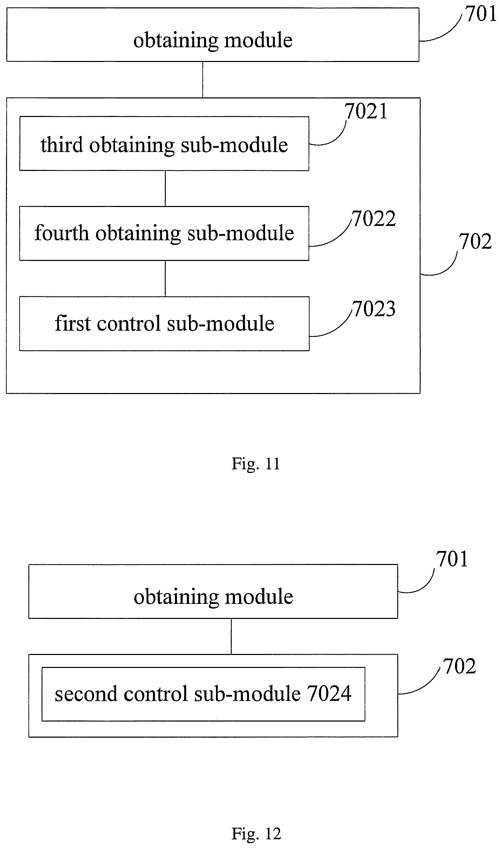

Fig. 11 is a block diagram of a control module according to an example embodiment of the present invention.

As shown in Fig. 11, in an embodiment, the control module 702 in Fig. 7 may include: a third obtaining sub-module 7021, a fourth obtaining sub-module 7022, and a first control sub-module 7023.

The third obtaining sub-module 7021 is configured to obtain a massage mode matched with the exercise information obtained by the obtaining module 701.

When the target massage equipment is controlled to operate according to the exercise information for providing a targeted massage for a user, a massage mode matched with the exercise information may be obtained firstly through the third obtaining sub-module 7021, i.e., a massage mode which matches with the exercise information and make the user's body be recovered from the fatigue due to exercise as soon as possible is obtained. For example, if the exercise equipment used by the user is a running machine, since the main body moving part is the leg and the leg conducts a large amount of activity, the massage mode may be a foot massage mode, a leg massage mode etc.; if the exercise equipment used by the user is a dumbbell or a horizontal bar, since the main body moving part is the arm and the arm conducts a large amount of activity, the massage mode may be an arm massage mode, a hand massage mode etc..

The fourth obtaining sub-module 7022 is configured to obtain the target massage equipment with the massage mode obtained by the third obtaining sub-module 7021.

For example, when the massage mode is foot massage mode, the target massage equipment may be a massage chair or a foot massager, a foot massage basin etc..

The first control sub-module 7023 is configured to control the target massage equipment obtained by the fourth obtaining sub-module 7022 to operate based on the massage mode.

Further, the target massage equipment with the massage mode is obtained by the fourth obtaining sub-module 7022, and then the target massage equipment is controlled by the first control sub-module 7023 to operate based on the massage mode so as to automatically provide the most comfortable, the most suitable and the most effective massage for the user with respect to the user's exercise information, such that the user's body may be recovered better and faster through the optimum massage after exercises, thereby prevent the user from selecting the massage mode manually and reducing the user's operations.

Since each massage mode has its corresponding preset massage parameter but each user's specific exercise information is distinct (for example, when the same exercise equipment is used to do exercise, the total exercise intensity, the exercise intensity of each body part, exercise ratio and length of exercise time may be changed), after the rough matched massage mode is determined, the target massage equipment may be controlled to operate followings.

The exercise information is compared with the preset exercise information corresponding to the massage mode.

The preset massage parameter corresponding to the massage mode is adjusted according to the comparison result, in which the preset massage parameter includes: one or more of a preset length of massage time and a preset massage intensity. For example, when the massage mode is upper limb massage, and the exercise intensity ratio of the upper limb to the lower limb in this exercise is higher than the preset exercise intensity ratio of the upper limb to the lower limb corresponding to the massage mode, the preset massage parameter corresponding to the massage mode may be increased for increasing the massage time and intensity for the upper limb; when the exercise intensity ratio of the upper limb to the lower limb in this exercise is lower than the preset exercise intensity ratio of the upper limb to the lower limb corresponding to the massage mode, the preset massage parameter corresponding to the massage mode may be decreased for decreasing the massage time and intensity for the upper limb. In this way, the inherent preset massage parameter of the massage mode may be adjusted according to user's specific exercise information, so as to provide a more intelligent targeted massage service for the user automatically. Thus, the user's body may be recovered from exercise better and faster, and the user's comfortable experience may be improved further.

In an embodiment, the massage mode includes: a historical massage mode matched with the exercise information.

When determining the massage mode, it is unnecessary to re-determine the mode every time. The exercise information of the user in each exercise and the determined matched massage mode may be stored in relation to each other. Thus, when the user does exercise next time, if the determined exercise information is exactly the same as that in one previous exercise, or the difference is minor, it is not required to re-determine the massage mode suitable for the user. Instead, the historical massage mode matched with the exercise information in this exercise may be directly determined as the massage mode for this exercise, so as to avoid re- determination and reduce operations and burden of the terminal and the target massage equipment.

Fig. 12 is a block diagram of another control module according to an example embodiment of the present invention.

As shown in Fig. 12, in an embodiment, the control module 702 in Fig. 7 may include: a second control sub-module 7024.

The second control sub-module 7024 is configured to control the target massage equipment to operate according to the exercise information by sending the exercise information to the target massage equipment.

When the subject of obtaining the exercise information is a terminal and the target massage equipment is controlled to operate according to the exercise information, the second control sub-module 7024 in the terminal may send the obtained exercise information to the target massage equipment, and then control the target massage equipment to operate according to the exercise information, such that the target massage equipment is controlled automatically through the terminal to provide the most comfortable, the most suitable and the most effective massage for the user with respect to the user's exercise information. Thus, after exercises, the user's body may be recovered better through the optimum massage, thereby prevent the user from selecting the massage mode manually and reducing the user's operations.

According to a third aspect of the present invention, a device for controlling a target massage equipment is provided. The device includes:
a processor;
a memory for storing an instruction executable by the processor;
in which the processor is configured to:
   obtain exercise information; and
   control the target massage equipment to operate according to the exercise information.

The processor is further configured to obtain exercise information by:
obtaining an arm swing frequency through a wearable device, in which the wearable device includes at least one of a smart band and a smart watch;
comparing the arm swing frequency with at least two preset arm swing frequencies; and if the arm swing frequency matches with any one of the at least two preset arm swing frequencies, determining the exercise information according to a moving part of a user's body corresponding to the any one of the at least two preset arm swing frequencies.

The processor is also configured to obtain exercise information, by:
obtaining a total exercise intensity when a user is doing exercise through a wearable device, in which the total exercise intensity includes one or more of a pulse rate, a pulse intensity, a heartbeat frequency and a heartbeat intensity when the user is doing exercise; and
determining the exercise information according to the total exercise intensity.

The processor is also configured to obtain exercise information, by:
determining an exercise equipment identification in the exercise information;
determining information of a used exercise equipment according to the exercise equipment identification, in which the information of the used exercise equipment includes: at least one of a name of the used exercise equipment name and a working parameter of the used exercise equipment.

The processor is also configured as: the exercise information including at least one of: body moving part information, an exercise intensity ratio between each two moving parts, exercise intensity information of each moving part and information of a used exercise equipment.

The processor is also configured to control the target massage equipment to operate according to the exercise information, by:
obtaining a massage mode matched with the exercise information;
obtaining the target massage equipment with the massage mode; and controlling the target massage equipment to operate based on the massage mode.

The processor is also configured as: the massage mode including: a historical massage mode matched with the exercise information.

The processor is also configured to control the target massage equipment to operate according to the exercise information by:
controlling the target massage equipment to operate according to the exercise information by sending the exercise information to the target massage equipment.

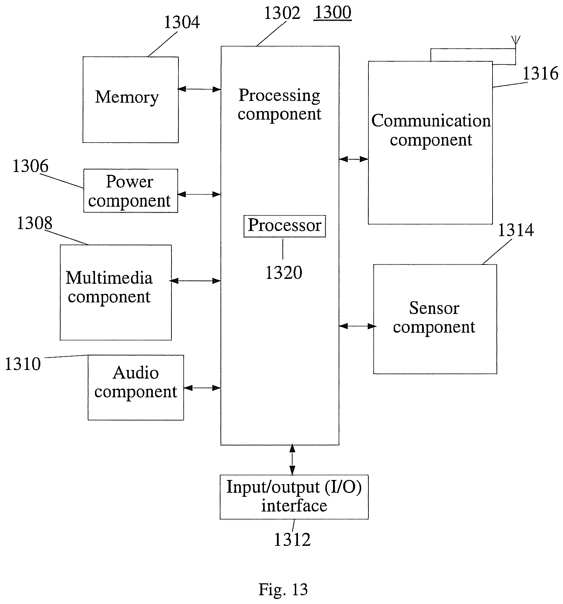

Fig. 13 is a block diagram of a device 1300 for controlling a target massage equipment according to an example embodiment of the present invention. The device is applied in a terminal. For example, the device 1300 may be a mobile phone, a computer a digital broadcast terminal, a messaging device, a gaming console, a tablet, a medical device, an exercise equipment, a personal digital assistant, and the like.

Referring to Fig. 13, the device 1300 may include one or at least two of the following components: a processing component 1302, a memory 1304, a power component 1306, a multimedia component 1308, an audio component 1310, an input/output (I/O) interface 1312, a sensor component 1314, and a communication component 1316.

The processing component 1302 typically controls overall operations of the device 1300, such as the operations associated with display, telephone calls, data communications, camera operations, and recording operations. The processing component 1302 may include one or at least two processors 1320 to execute instructions to perform all or part of the acts in the above described methods. Moreover, the processing component 1302 may include one or at least two modules which facilitate the interaction between the processing component 1302 and other components. For instance, the processing component 1302 may include a multimedia module to facilitate the interaction between the multimedia component 1308 and the processing component 1302 .

The memory 1304 is configured to store various types of data to support the operation of the device 1300. Examples of such data include instructions for any applications or methods operated on the device 1300, contact data, phonebook data, messages, pictures, video, etc. The memory 1304 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 1306 provides power to various components of the device 1300. The power component 1306 may include a power management system, one or more power sources, and any other components associated with the generation, management, and distribution of power in the device 1300.

The multimedia component 1308 includes a screen providing an output interface between the device 1300 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action. In some embodiments, the multimedia component 1308 includes a front camera and/or a rear camera. The front camera and the rear camera may receive an external multimedia datum while the device 1300 is in an operation mode, such as a photographing mode or a video mode. Each of the front camera and the rear camera may be a fixed optical lens system or have focus and optical zoom capability.

The audio component 1310 is configured to output and/or input audio signals. For example, the audio component 1310 includes a microphone ("MIC") configured to receive an external audio signal when the device 1300 is in an operation mode, such as a call mode, a recording mode, and a voice recognition mode. The received audio signal may be further stored in the memory 1304 or transmitted via the communication component 1316. In some embodiments, the audio component 1310 further includes a speaker to output audio signals.

The I/O interface 1312 provides an interface between the processing component 1302 and peripheral interface modules, such as a keyboard, a click wheel, buttons, and the like. The buttons may include, but are not limited to, a home button, a volume button, a starting button, and a locking button.

The sensor component 1314 includes one or at least two sensors to provide status assessments of various aspects of the device 1300. For instance, the sensor component 1314 may detect an open/closed status of the device 1300, relative positioning of components, e.g., the display and the keypad, of the device 1300, a change in position of the device 1300or a component of the device 1300, a presence or absence of user contact with the device 1300, an orientation or an acceleration/deceleration of the device 1300, and a change in temperature of the device 1300. The sensor component 1314 may include a proximity sensor configured to detect the presence of nearby objects without any physical contact. The sensor component 1314 may also include a light sensor, such as a CMOS or CCD image sensor, for use in imaging applications. In some embodiments, the sensor component 1314 may also include an accelerometer sensor, a gyroscope sensor, a magnetic sensor, a pressure sensor, or a temperature sensor.

The communication component 1316 is configured to facilitate communication, wired or wirelessly, between the device 1300 and other devices. The device 1300 can access a wireless network based on a communication standard, such as WiFi, 2G, or 3G, or a combination thereof. In one exemplary embodiment, the communication component 1316 receives a broadcast signal or broadcast associated information from an external broadcast management system via a broadcast channel. In one exemplary embodiment, the communication component 1316 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the device 1300 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods.

In exemplary embodiments, there is also provided a non-transitory computer-readable storage medium including instructions, such as included in the memory 1304, executable by the processor 1320 in the device 1300, for performing the above-described methods. For example, the non-transitory computer-readable storage medium may be a ROM, a RAM, a CD-ROM, a magnetic tape, a floppy disc, an optical data storage device, and the like.

A non-transitory computer-readable storage medium has stored therein instructions that, when executed by a processor, causes the device 1300 to perform the method for controlling a target massage equipment, including:
obtaining exercise information; and
controlling the target massage equipment to operate according to the exercise information.

In an embodiment, obtaining exercise information includes:
obtaining an arm swing frequency through a wearable device, in which the wearable device includes at least one of a smart band and a smart watch;
comparing the arm swing frequency with at least two preset arm swing frequencies; and
when the arm swing frequency matches with any one of the at least two preset arm swing frequencies, determining the exercise information according to a moving of a user's body part corresponding to the any one of the at least two preset arm swing frequencies.

In an embodiment, obtaining exercise information includes:
obtaining a total exercise intensity when a user is doing exercise through a wearable device, in which the total exercise intensity includes one or more of a pulse rate, a pulse intensity, a heartbeat frequency and a heartbeat intensity when the user is doing exercise; and
determining the exercise information according to the total exercise intensity.

In an embodiment, obtaining exercise information includes:
determining an exercise equipment identification in the exercise information;
determining information of a used exercise equipment according to the exercise equipment identification, in which the information of the used exercise equipment includes: at least one of a name of the used exercise equipment and a working parameter of the used exercise equipment.

In an embodiment, the exercise information includes at least one of: body moving part information, an exercise intensity ratio between each two moving parts, exercise intensity information of each moving part and information of a used exercise equipment.

In an embodiment, controlling the target massage equipment to operate according to the exercise information includes:
obtaining a massage mode matched with the exercise information;
obtaining the target massage equipment with the massage mode; and controlling the target massage equipment to operate based on the massage mode.

In an embodiment, the massage mode includes: a historical massage mode matched with the exercise information.

In an embodiment, controlling the target massage equipment to operate according to the exercise information includes:
controlling the target massage equipment to operate according to the exercise information by sending the exercise information to the target massage equipment.

Other embodiments of the present invention will be apparent to those skilled in the art from consideration of the specification and practice of the invention disclosed here. This application is intended to cover any variations, uses, or adaptations of the invention following the general principles thereof and including such departures from the present invention as come within known or customary practice in the art.

## Claims

1. A device for controlling a target massage equipment, comprising:
an obtaining module (701), configured to obtain exercise information, wherein the obtaining module (701) comprises at least one of a first obtaining sub-module (7011) and a second obtaining sub-module (7014), the first obtaining sub-module (7011) is configured to obtain an arm swing frequency through a wearable device, and the second obtaining sub-module (7014) is configured to obtain a total exercise intensity when a user is doing exercise through the wearable device; and
a control module (702), configured to control the target massage equipment to operate according to the exercise information obtained by the obtaining module (701) and wherein the wearable device comprises at least one of a smart band and a smart watch.

2. The device according to claim 1, wherein the obtaining module (701) comprises:
a comparing sub-module (7012), configured to compare the arm swing frequency obtained by the first obtaining sub-module (7011) with at least two preset arm swing frequencies; and
a first determining sub-module (7013), configured to determine the exercise information according to a moving part of a user's body corresponding to any one of the at least two preset arm swing frequencies when a comparison result obtained by the comparing sub-module (7012) indicates that the arm swing frequency matches with the any one of the at least two preset arm swing frequencies.

3. The device according to claim 1, wherein the obtaining module (701) comprises:
a second determining sub-module (7015), configured to determine the exercise information according to the total exercise intensity obtained by the second obtaining sub-module (7014),
in which the total exercise intensity comprises one or more of a pulse rate, a pulse intensity, a heartbeat frequency and a heartbeat intensity when the user is doing exercise.

4. The device according to claim 1, wherein the obtaining module (701) comprises:
a third determining sub-module (7016), configured to determine an exercise equipment identification in the exercise information;
a fourth determining sub-module (7017), configured to determine information of a used exercise equipment according to the exercise equipment identification determined by the third determining sub-module (7016), in which the information of the used exercise equipment comprises: at least one of a name of the used exercise equipment and a working parameter of the used exercise equipment.

5. The device according to claim 1, wherein the exercise information comprises at least one of: body moving part information, an exercise intensity ratio between each two moving parts, exercise intensity information of each moving part and information of a used exercise equipment.

6. The device according to any of claims 1-5, wherein the control module (702) comprises:
a third obtaining sub-module (7021), configured to obtain a massage mode matched with the exercise information obtained by the obtaining module;
a fourth obtaining sub-module (7022), configured to obtain the target massage equipment with the massage mode obtained by the third obtaining sub-module (7021); and
a first control sub-module (7023), configured to control the target massage equipment obtained by the fourth obtaining sub-module (7022) to operate based on the massage mode.

7. The device according to claim 6, wherein the massage mode comprises: a historical massage mode matched with the exercise information.

## Patentansprüche

1. Vorrichtung zum Steuern eines Zielmassagegeräts, umfassend:
ein Erhaltungsmodul (701), das dazu ausgestaltet ist, Trainingsinformationen zu erhalten, wobei das Erhaltungsmodul (701) mindestens eines von einem ersten Erhaltungsuntermodul (7011) und einem zweiten Erhaltungsuntermodul (7014) umfasst, wobei das erste Erhaltungsuntermodul (7011) dazu ausgestaltet ist, eine Armschwungfrequenz mittels einer tragbare Vorrichtung zu erhalten, und das zweite Erhaltungsuntermodul (7014) dazu ausgestaltet ist, eine Gesamttrainingsintensität mittels der tragbaren Vorrichtung zu erhalten, wenn ein Benutzer trainiert, und
ein Steuermodul (702), das dazu ausgestaltet ist, das Zielmassagegerät zu steuern, um gemäß den Trainingsinformationen zu arbeiten, die von dem Erhaltungsmodul (701) erhalten werden, und wobei die tragbare Vorrichtung mindestens eines von einem Smartband und einer Smartwatch umfasst.

2. Vorrichtung nach Anspruch 1, wobei das Erhaltungsmodul (701) umfasst:
ein Vergleichsuntermodul (7012), das dazu ausgestaltet ist, die Armschwungfrequenz, die von dem ersten Erhaltungsuntermodul (7011) erhalten wird, mit mindestens zwei voreingestellten Armschwungfrequenzen zu vergleichen,
und
ein erstes Bestimmungsuntermodul (7013), das dazu ausgestaltet ist, die Trainingsinformationen gemäß einem sich bewegenden Körperteil eines Benutzers entsprechend einer der mindestens zwei voreingestellten Armschwungfrequenzen zu bestimmen, wenn ein von dem Vergleichsuntermodul (7012) erhaltenes Vergleichsergebnis anzeigt, dass die Armschwungfrequenz mit der einen der mindestens zwei voreingestellten Armschwungfrequenzen übereinstimmt.

3. Vorrichtung nach Anspruch 1, wobei das Erhaltungsmodul (701) umfasst:
ein zweites Bestimmungsuntermodul (7015), das dazu ausgestaltet ist, die Trainingsinformationen gemäß der Gesamttrainingsintensität zu bestimmen, die von dem zweiten Erhaltungsuntermodul (7014) erhalten wird,
wobei die Gesamttrainingsintensität eines oder mehrere von einer Pulsrate, einer Pulsintensität, einer Herzfrequenz und einer Herzschlagintensität, wenn der Benutzer trainiert, umfasst.

4. Vorrichtung nach Anspruch 1, wobei das Erhaltungsmodul (701) umfasst:
ein drittes Bestimmungsuntermodul (7016), das dazu ausgestaltet ist, in den Trainingsinformationen eine Trainingsgeräteidentifikation zu bestimmen,
ein viertes Bestimmungsuntermodul (7017), das dazu ausgestaltet ist, Informationen über ein verwendetes Trainingsgerät gemäß der von dem dritten Bestimmungsuntermodul (7016) bestimmten Trainingsgeräteidentifikation zu bestimmen, wobei die Informationen über das verwendete Trainingsgerät umfassen: mindestens eines von einer Bezeichnung des verwendeten Trainingsgeräts und einem Arbeitsparameter des verwendeten Trainingsgeräts.

5. Vorrichtung nach Anspruch 1, wobei die Trainingsinformationen mindestens eines umfassen von: Informationen über sich bewegende Körperteile, ein Trainingsintensitätsverhältnis zwischen jeweils zwei sich bewegenden Teilen, Trainingsintensitätsinformationen jedes sich bewegenden Teils und Informationen über ein verwendetes Trainingsgerät.

6. Vorrichtung nach einem der Ansprüche 1 - 5, wobei das Steuermodul (702) umfasst:
ein drittes Erhaltungsuntermodul (7021), das dazu ausgestaltet ist, einen Massagemodus zu erhalten, der mit den durch das Erhaltungsmodul erhaltenen Trainingsinformationen übereinstimmt,
ein viertes Erhaltungsuntermodul (7022), das dazu ausgestaltet ist, das Zielmassagegerät mit dem von dem dritten Erhaltungsuntermodul (7021) erhaltenen Massagemodus zu erhalten, und
ein erstes Steueruntermodul (7023), das dazu ausgestaltet ist, das von dem vierten Erhaltungsuntermodul (7022) erhaltene Zielmassagegerät zu steuern, um basierend auf dem Massagemodus zu arbeiten.

7. Vorrichtung nach Anspruch 6, wobei der Massagemodus umfasst: einen historischen Massagemodus, der auf die Trainingsinformationen abgestimmt ist.

## Revendications

1. Dispositif permettant de commander un équipement de massage cible, comprenant :
un module d'obtention (701) configuré pour obtenir des informations d'exercice, dans lequel le module d'obtention (701) comprend un premier sous-module d'obtention (7011) et/ou un deuxième sous-module d'obtention (7014), le premier sous-module d'obtention (7011) est configuré pour obtenir une fréquence de balancement des bras par le biais d'un dispositif prêt à porter, et le deuxième sous-module d'obtention (7014) est configuré pour obtenir une intensité totale d'exercice lorsqu'un utilisateur fait de l'exercice, par le biais du dispositif prêt à porter ; et
un module de commande (702) configuré pour commander l'équipement de massage cible afin qu'il fonctionne selon les informations d'exercice obtenues par le module d'obtention (701) et dans lequel le dispositif prêt à porter comprend un bracelet connecté et/ou une montre connectée.

2. Dispositif selon la revendication 1, dans lequel le module d'obtention (701) comprend :
un sous-module de comparaison (7012) configuré pour comparer la fréquence de balancement des bras, obtenue par le premier sous-module d'obtention (7011), à au moins deux fréquences prédéfinies de balancement des bras ; et
un premier sous-module de détermination (7013) configuré pour déterminer les informations d'exercice selon une partie en mouvement du corps de l'utilisateur, correspondant à l'une quelconque des au moins deux fréquences prédéfinies de balancement des bras, lorsqu'un résultat de comparaison obtenu par le sous-module de comparaison (7012) indique que la fréquence de balancement des bras concorde avec celle quelconque des au moins deux fréquences prédéfinies de balancement des bras.

3. Dispositif selon la revendication 1, dans lequel le module d'obtention (701) comprend :
un deuxième sous-module de détermination (7015) configuré pour déterminer les informations d'exercice selon l'intensité totale d'exercice, obtenue par le deuxième sous-module d'obtention (7014),
dans lequel l'intensité totale d'exercice comprend une fréquence de pouls et/ou une intensité de pouls et/ou une fréquence des battements cardiaques et/ou une intensité des battements cardiaques lorsque l'utilisateur fait de l'exercice.

4. Dispositif selon la revendication 1, dans lequel le module d'obtention (701) comprend :
un troisième sous-module de détermination (7016) configuré pour déterminer une identification d'équipement d'exercice dans les informations d'exercice ;
un quatrième sous-module de détermination (7017) configuré pour déterminer des informations sur un équipement d'exercice utilisé, en fonction de l'identification d'équipement d'exercice, déterminée par le troisième sous-module de détermination (7016), dans lequel les informations sur l'équipement d'exercice utilisé comprennent : un nom de l'équipement d'exercice utilisé et/ou un paramètre de fonctionnement de l'équipement d'exercice utilisé.

5. Dispositif selon la revendication 1, dans lequel les informations d'exercice comprennent : des informations sur une partie en mouvement et/ou un rapport d'intensité d'exercice entre chaque partie d'une paire de parties en mouvement et/ou des informations d'intensité d'exercice sur chaque partie en mouvement et/ou des informations sur un équipement d'exercice utilisé.

6. Dispositif selon l'une quelconque des revendications 1 à 5, dans lequel le module de commande (702) comprend :
un troisième sous-module d'obtention (7021) configuré pour obtenir un mode de massage concordant avec les informations d'exercice obtenues par le module d'obtention ;
un quatrième sous-module d'obtention (7022) configuré pour obtenir l'équipement de massage cible à l'aide du mode de massage obtenu par le troisième sous-module d'obtention (7021) ; et
un premier sous-module de commande (7023) configuré pour commander l'équipement de massage cible obtenu par le quatrième sous-module d'obtention (7022) afin qu'il fonctionne sur la base du mode de massage.

7. Dispositif selon la revendication 6, dans lequel le mode de massage comprend : un mode de massage antérieur concordant avec les informations d'exercice.
